# FASCICULE DE BREVET EUROPEEN

(11) **EP 3 352 775 B1**
(45) Date de publication et mention de la délivrance du brevet: **01.11.2023**
(21) Numéro de dépôt: 16775555.2
(22) Date de dépôt: 21.09.2016
(51) Int. Cl.: A61K 38/12, C07K 14/705, C07K 5/12, C07K 7/64, A61P 15/08, A61K 8/64

(54) **UTILISATION D'UN TRIPEPTIDE CYCLIQUE POUR AMELIORER LE METABOLISME ENERGETIQUE CELLULAIRE**
VERWENDUNG EINES CYCLISCHEN TRIPEPTIDS ZUR VERBESSERUNG DES ZELLENERGIESTOFFWECHSELS
USE OF A CYCLIC TRIPEPTIDE FOR IMPROVING CELLULAR ENERGY METABOLISM

(30) Priorité: 21.09.2015 FR 1558899
(43) Date de publication de la demande: 01.08.2018
(73) Titulaire: Assistance Publique - Hôpitaux de Paris, 75004 Paris (FR); Université Paris Cité, 75006 Paris (FR); Institut National de la Santé et de la Recherche Médicale (INSERM), 75013 Paris (FR); Centre National de la Recherche Scientifique, 75016 Paris (FR)
(72) Inventeur: WOLF, Jean-Philippe, 75013 Paris (FR); LOMBÈS, Anne, 75013 Paris (FR); BOMSEL, Morgane, 75003 Paris (FR)
(74) Mandataire: Icosa
(86) Numéro de dépôt international: PCT/EP2016/072476
(87) Numéro de publication internationale: WO 2017/050854

(56) Documents cités:
- WO-A2-2005/051799
- A. ZIYYAT ET AL: "Cyclic FEE peptide increases human gamete fusion and potentiates its RGD-induced inhibition", HUMAN REPRODUCTION, vol. 20, no. 12, 11 août 2005 (2005-08-11) , pages 3452-3458, XP055291445, GB ISSN: 0268-1161, DOI: 10.1093/humrep/dei241
- R. ROTIVAL ET AL: "Comprehensive determination of the cyclic FEE peptide chemical stability in solution", JOURNAL OF PHARMACEUTICAL AND BIOMEDICAL ANALYSIS, vol. 89, 29 octobre 2013 (2013-10-29), pages 50-55, XP055291515, US ISSN: 0731-7085, DOI: 10.1016/j.jpba.2013.10.026
- BARRAUD-LANGE V ET AL: "Cyclic QDE peptide increases fertilization rates and provides healthy pups in mouse", FERTILITY AND STERILITY, ELSEVIER SCIENCE INC, NEW YORK, NY, USA, vol. 91, no. 5, 1 mai 2009 (2009-05-01), pages 2110-2115, XP026057612, ISSN: 0015-0282, DOI: 10.1016/J.FERTNSTERT.2008.05.088 [extrait le 2008-08-09]
- Anonymous: "Infertilité masculine : la recherche progresse", , 26 August 2015 (2015-08-26), pages 1-3, XP055811407, Retrieved from the Internet: URL:https://www.allodocteurs.fr/grossesse- enfant/procreation/fertilite-infertilite/i nfertilite-masculine-la-recherche-progress e_9214.html [retrieved on 2021-06-08]
- Maxime Vautier ET AL: "Infertilité masculine : la recherche progresse", Youtube, 26 August 2015 (2015-08-26), page 1 pp., XP54981884, Retrieved from the Internet: URL:https://www.allodocteurs.fr/media/cds_ infertilite_masculine_recherche_081112_big .mp4 [retrieved on 2021-06-09]
- Anonymous: "Improvement of IVF Fertilization Rates, by the Cyclic Tripeptide FEE - Prospective Randomized Study", ClinicalTrials.gov, 12 June 2014 (2014-06-12), pages 1-6, XP055812435, Retrieved from the Internet: URL:https://clinicaltrials.gov/ct2/show/NC T02161861 [retrieved on 2021-06-10]

## Description

La présente invention concerne le domaine de l'assistance médicale à la procréation (AMP) et plus généralement toutes les applications médicales, vétérinaires ou autres dans lesquelles une stimulation de l'activité mitochondriale, ou plus généralement énergétique, des cellules est souhaitée, en particulier lors de protocoles comportant une étape de culture cellulaire ou de maintien de cellules *ex vivo.*

Plus de 15% des couples ont recours à l'Assistance Médicale à la Procréation au cours de leur vie génitale. Lorsque que le sperme est altéré, il est fréquent de recourir aux techniques de microinjection pour obtenir des fécondations. Cette technique est très invasive pour l'ovocyte. Par ailleurs, lors de fécondations *in vitro* (FIV) avec sperme normal, on retrouve régulièrement 3 à 5% d'échecs de fécondation inexpliqués.

Dans une demande de brevet antérieure (WO 2005/051799 A2), l'équipe des inventeurs a décrit un tripeptide cyclique (Phe, Ac Glu, Ac Glu) de formule C-S-F-E-E-C (SEQ ID No : 1) avec une liaison de cyclisation entre les deux cystéines terminales (FEEc), ainsi que son action augmentant les capacités fécondantes des gamètes humains. Il existe des équivalents de la molécule dans les différentes espèces animales ayant les mêmes propriétés et également décrits dans ce document.

Poursuivant leurs recherches, les inventeurs ont mis en évidence que la molécule FEEc a un impact sur les spermatozoïdes (exemple 1 ci-dessous). En particulier, elle améliore les paramètres du mouvement spermatique tels qu'analysés par CASA *(Computer Aided Sperm Analysis*)*.* Ainsi, par exemple, la vitesse linéaire et l'amplitude du débattement latéral de la tête sont respectivement augmentés de 7 et 8 % (P<0,05 et P<0.002) (Exemple 1). Il en résulte une augmentation de près de 30% du pourcentage de spermatozoïdes hyperactivés (P<0,009). Ce sont ces spermatozoïdes hyperactivés qui sont les spermatozoïdes fécondants. Pour que le spermatozoïde augmente sa vitesse de progression, il est logique de penser qu'il augmente sa consommation d'ATP ou du moins que son métabolisme énergétique est amélioré, puisque son mouvement est créé par des liaisons de bras de dynéines aux tubules voisins de l'axonème. Les dynéines sont des ATPases. En étudiant le métabolisme mitochondrial des spermatozoïdes exposés au FEEc, les inventeurs ont émis l'hypothèse que celui-ci induit une augmentation du potentiel de membrane mitochondrial, témoin soit d'une augmentation de la synthèse d'ATP par les mitochondries, soit d'une moindre consommation. Ainsi, le FEEc améliore le métabolisme énergétique mitochondrial ou plus généralement le métabolisme énergétique cellulaire des spermatozoïdes, soit en améliorant la production d'ATP, soit en rationnalisant son utilisation par la cellule.

La présente description porte donc, de manière générale, sur l'utilisation d'un peptide cyclique comprenant le tripeptide reproduisant un site de liaison de la Fertiline béta à l'intégrine de l'ovocyte ou d'un tripeptide de formule EEP (SEQ ID No : 2), pour son utilisation comme médicament pour améliorer l'activité mitochondriale ou plus généralement pour améliorer le métabolisme énergétique cellulaire. Les applications d'un tel peptide sont ici illustrées sur deux types cellulaires différents que sont le spermatozoïde et l'ovocyte et sur le développement d'un organisme multicellulaire qu'est un embryon. Les résultats présentés dans la partie expérimentale supportent le fait de pouvoir utiliser ce peptide dans d'autres applications dans lesquelles une amélioration du métabolisme énergétique cellulaire est souhaitée. En particulier, ce peptide peut être utile pour améliorer le rendement des cultures cellulaires, notamment des lymphocytes. Dans un mode de mise en oeuvre particulier, le peptide est vectorisé, c'est-à-dire administré en association avec un agent, une molécule, une composition ou tout autre type de vecteur qui facilite son entrée dans les cellules. Plus généralement, la vectorisation sert à moduler et contrôler la distribution d'un principe actif vers une cible en l'associant à un vecteur.

Dans ce qui précède, le "peptide cyclique comprenant le tripeptide reproduisant un site de liaison de la fertiline béta à l'intégrine de l'ovocyte" correspond au peptide décrit dans la demande de brevet WO 2005/051799 A2 mentionnée ci-dessus. Comme mentionné dans cette demande antérieure, notamment dans le Tableau 1, le tripeptide varie selon les espèces. Il peut être cyclisé par tout moyen connu de l'homme du métier, en particulier par l'intermédiaire de deux résidus cystéines situés de part et d'autre du tripeptide. De manière générale, toutes les déclinaisons décrites dans la demande WO 2005/051799 A2 sont considérées comme répondant à la définition du "peptide cyclique comprenant le tripeptide formant un site de liaison de la fertiline béta à l'intégrine de l'ovocyte". Pour faciliter la lecture du présent texte, ce peptide cyclique, ainsi que le médicament le contenant à titre de principe actif, seront désigné ici par la formule "FEEc". L'homme du métier comprendra parfaitement que cette notation couvre également les formes utilisables chez d'autres espèces que l'homme, telles que, par exemple, le tripeptide cyclique TDE qui devrait être utilisé chez les bovins.

On comprendra à la lecture de la description détaillée qui suit ainsi que des exemples, que l'utilisation du peptide cyclique ci-dessus ne se limite pas aux applications en procréation médicalement assistées mais qu'elle ouvre de réelles perspectives dans beaucoup d'autres domaines. Ainsi, la description concerne plus généralement l'utilisation du FEEc dans les applications médicales ou non médicales dans lesquelles une stimulation du métabolisme énergétique cellulaire est souhaitée.

### Action sur les spermatozoïdes en Insémination Intra Utérine

En améliorant les paramètres du mouvement spermatique, le FEEc est également susceptible d'améliorer les taux de grossesses en Insémination Intra Utérine (IIU). Selon un premier aspect particulier, le FEEc est utilisé pour augmenter la vitesse de progression des spermatozoïdes dans un protocole de procréation médicalement assistée (PMA). Toujours dans le cadre d'une AMP, le FEEc peut être utilisé pour améliorer les paramètres du mouvement des spermatozoïdes et augmenter le taux de spermatozoïdes hyperactivés. Aussi, l'utilisation du FEEc est particulièrement intéressante dans un protocole d'insémination intra utérine (IIU), tant chez l'homme que chez des mammifères non humains. Lors de la mise en oeuvre de cet aspect, les spermatozoïdes sont de préférence incubés pendant une minute à 3 heures en présence de 10 à 100 µM de peptide puis lavés avant l'insémination intra utérine.

### Action sur la maturation de l'ovocyte in vitro

La molécule est également efficace sur les ovocytes. La maturation *in vitro* des ovocytes humains bloqués en vésicule germinale passe de 37.71 à 59,30% (P<5,7 10⁻⁵) en présence de FEEc (exemple 2). Chez les patientes âgées de 37 ans et plus, ce taux passe de 36,96% à 68,29%,(P<0,003) ce qui montre que la molécule est particulièrement efficace dans cette tranche d'âge. Les ovocytes des femmes de 37 à 40 ans sont aneuploïdes dans au moins 50% et plus généralement dans environ 80% des cas du fait d'une chute de leur activité mitochondriale. La molécule est donc susceptible d'améliorer la ploïdie des ovocytes et par là même celle des embryons dont le potentiel de développement et la capacité implantatoire dépend également de l'activité mitochondriale de l'ovocyte qui a été fécondé. L'augmentation significative des taux de grossesse chez les femmes de moins de 37 ans, dans le cadre de l'étude clinique « Fertiline » décrite ci-dessous, montre que cet effet bénéfique a lieu sur tout ovocyte et notamment lors de sa fécondation et du développement embryonnaire précoce. L'hypothèse d'après ces résultats et que la fertiline est à même d'améliorer la ploïdie ovocytaire.

La supplémentation des milieux de culture et des milieux de fécondation *in vitro,* et des milieux d'incubation des embryons, avec la molécule permet donc d'améliorer la maturation ovocytaire et embryonnaire (notamment pour les femmes moins de 30 ans et de 37 ans et plus) et le taux de fécondation en FIV classique, ainsi qu'en FIV avec ICSI (injection intracytoplasmique de spermatozoïde).

La supplémentation des milieux de culture lors de la fécondation *in vitro,* avec ou sans micromanipulation, avec la molécule FEEc permet donc d'améliorer le taux de grossesse et d'enfants nés, en particulier chez les femmes de moins de 37 ans dans les conditions expérimentales mises en oeuvre.

### Action dans les protocoles de Maturation In Vitro de l'ovocyte (MIV)

La molécule FEEc est aussi susceptible d'être efficace dans les protocoles de maturation *in vitro* des ovocytes pour la préservation de la fertilité (MIV).

Dans un protocole de fécondation *in vitro,* la maturation de l'ovocyte est en général achevée lors du recueil des ovocytes. Toutefois, il arrive que des ovocytes soient encore immatures. En outre, certaines femmes présentent des anomalies de la fonction ovarienne ou un état clinique qui rendent les stimulations difficiles. Les ponctions ont alors volontairement lieu à un stade immature pour une maturation *in vitro.* Le peptide pourrait efficacement aider à cette maturation (maturation *in vitro* pour la préservation de la fertilité).

L'ovocyte totalement immature présente un grand noyau appelé vésicule germinale (VG). L'ovocyte mature se caractérise par la présence du 1^{er} globule polaire (GP) dans l'espace périvitellin (entre la surface de l'ovocyte et la zone pellucide). Seuls les ovocytes matures sont fécondables.

Les inventeurs ont mis en évidence que le FEEc permet d'améliorer la maturation des ovocytes *in vitro.* Ceci s'explique par l'effet du tripeptide sur l'activité mitochondriale ou sur le métabolisme énergétique de l'ovocyte. En effet, l'activité mitochondriale ou plus généralement le métabolisme énergétique diminue avec l'âge, et la maturation de l'ovocyte comporte plusieurs étapes très consommatrices d'énergie :
- Rupture de la Vésicules Germinale
- Condensation des Chromosomes
- Formation de la plaque Métaphasique
- Formation du Fuseau
- Synthèse des protéines de Check-Point
- Télophase
- Expulsion du GP.

Or ces troubles de la maturation ovocytaire qui s'aggravent avec l'âge sont corrigés par la microinjection de mitochondries provenant de cellules jeunes (ovocytes de donneuses jeunes ou cellules souches ovogoniales). Cela renforce la probabilité selon laquelle le FEEc corrige effectivement le défaut cellulaire lié à une insuffisance mitochondriale ou plus généralement énergétique.

Selon un autre de ses aspects, la présente invention porte donc sur l'utilisation du FEEc pour améliorer la maturation *in vitro* d'un ovocyte. L'amélioration de la qualité de la méiose est probablement à l'origine de la baisse du taux de fausses couches observée également chez des femmes plus jeunes, si bien que cet aspect de l'invention est également intéressant pour améliorer la maturation in vitro des ovocytes de femmes de moins de 37 ans, voire de moins de 30 ans. Dans la mise en oeuvre de cet aspect de l'invention, l'ovocyte est incubé pendant une durée comprise entre 1 heure et 4 jours, en particulier jusqu'à 3 jours ou 24 heures, et en présence de 10 à 100 µM de peptide.

### Action sur l'activation de l'ovocyte fécondé

Les inventeurs ont également mis en évidence une augmentation de la décondensation de la tête des spermatozoïdes après fécondation. Cela traduit une amélioration de l'activation de l'ovocyte lors de la fécondation. Celle-ci est d'ailleurs liée à l'activité mitochondriale de l'ovocyte.

### Action sur la blastoformation

Les inventeurs ont également mis en évidence (chez la souris) que le FEEc permet une amélioration de la blastoformation. Ceci peut également être attribué à l'effet du tripeptide sur l'activité mitochondriale ou plus généralement sur le métabolisme de la cellule. En effet, il est connu qu'il n'y a pas de réplication d'ADN mitochondrial pendant l'embryogénèse préimplantatoire. Pendant la première semaine de développement, le zygote (ou oeuf) se divise par mitoses successives en commençant par 2, puis 4 cellules, en passant par le stade de morula jusqu'à atteindre le stade de blastocyste, en utilisant préférentiellement les mitochondries initialement présentes dans l'ovocyte. Un déficit mitochondrial (en nombre ou en rendement) peut donc être à l'origine d'une instabilité chromosomique des blastomères lors de la méiose et des mitoses, et conduire à un arrêt de l'évolution du zygote, de l'embryon, voire de la grossesse. Ceci est une cause fréquente de fausse couche spontanée après fécondation naturelle ou après transfert des embryons lors d'une FIV.

La présente description porte donc également sur l'utilisation du FEEc pour améliorer la ploïdie des blastomères pendant la première semaine de développement du zygote. De ce fait, la description concerne l'utilisation de FEEc pour diminuer le nombre de fausses couches. La description concerne aussi l'utilisation de FEEc pour diminuer le risque d'aneuploïdie, en particulier de trisomie. Dans la mise en oeuvre de cet aspect, l'embryon est incubé pendant une durée comprise entre 24 heures et 6 ou 7 jours en présence de 10 à 100 µM de peptide.

Les utilisations décrites ci-dessus sont particulièrement utiles dans un protocole de fécondation *in vitro* (FIV). Chez l'humain, elles permettent à des femmes de tout âge, d'avoir des enfants par AMP avec leurs propres ovocytes sans recourir aux injections de mitochondries décrites dans la littérature (effet majeur le plus documenté jusqu'à présent).

### Diminution des risques de fausses couches

Le FEEc peut également être utilisé pour diminuer les risques de fausse couche, comme cela a été mentionnée précédemment et est illustré dans la partie expérimentale ci-dessous.

### Diminution des risques de trisomie

Le FEEc peut également être utilisé pour diminuer les risques de trisomie, ou plus généralement d'aneuploïdie, lors d'une FIV, chez toutes les femmes, notamment chez des femmes de 35, 36, 37, 38, 39 ou 40 ans et plus.

### Amélioration de la cinétique de développement de l'embryon in vitro

La présente description concerne également l'utilisation du FEEc pour améliorer le développement embryonnaire pré-implantatoire *in vitro.* Dans un aspect particulier, le développement pré-implantatoire est obtenu en condition de culture prolongée. L'amélioration de la cinétique du développement de l'embryon permet d'améliorer le taux de naissance.

### Action en reproduction naturelle

Bien que les effets du FEEc sur l'ovocyte et le zygote aient été démontrés par les inventeurs dans un contexte de FIV, il est évident que ces effets pourraient être obtenus également lors des fécondations naturelles, par exemple par administration, au moment de l'ovulation, de FEEc par voie vaginale, le tripeptide étant couplé à des moyens aptes à le vectoriser jusqu'à l'ovocyte.

### Action lors de la cryopréservation des gamètes et des embryons

Il a également été montré que le taux de survie des ovocytes cryopréservés dépend entre autre de leur activité mitochondriale. Il est probable qu'il en soit de même pour les embryons. Le peptide FEEc est donc susceptible d'améliorer les taux de survie et/ou la qualité des gamètes et embryons cryopréservés lors de leur décongélation.

### Action sur d'autres types cellulaires

Le tripeptide cyclique a été fabriqué pour se lier à l'intégrine α6β1 sur l'ovocyte. Il est capable de se lier à la membrane cytoplasmique de différents autres types cellulaires car cette intégrine est très ubiquitaire. Cette molécule est donc vraisemblablement capable d'augmenter l'activité énergétique de nombreux types cellulaires. Elle est donc susceptible d'utilisations multiples en dehors de la FIV.

Le mode d'action de la molécule se faisant probablement par l'intermédiaire de l'intégrine α6β1, le FEEc est susceptible d'avoir des effets sur beaucoup d'autres types cellulaires. Cette molécule pourrait être utilisée pour améliorer le rendement de toute culture cellulaire.

Comme mentionné plus haut, le FEEc agit possiblement par l'intermédiaire de l'intégrine α6β1 ou d'un autre récepteur, présent sur de nombreux types cellulaires. Il peut donc être utilisé pour améliorer l'activité mitochondriale ou le métabolisme énergétique de toute cellule porteuse de cette intégrine ou de cet autre récepteur. Une application immédiate de cette propriété est l'amélioration du rendement de toute culture cellulaire. La présente description porte donc également sur un procédé d'amélioration de l'activité mitochondriale ou plus généralement énergétique de cellules *in vitro,* comportant une étape de mise en présence des cellules en question avec le FEEc. Ce procédé peut avantageusement être mis en oeuvre sur des cellules primaires cultivées *ex vivo* dans l'optique d'être administrées à un patient dans le cadre d'une thérapie cellulaire. A titre d'exemples non limitatifs de cultures cellulaires susceptibles de bénéficier de ce procédé, on peut citer les cultures de peau en vue de greffes de peau, les cultures de lymphocytes en vue d'immunothérapies cellulaires, *etc.*

La description concerne l'utilisation d'un peptide FEEc dans le but de favoriser la culture *ex vivo* de tous types cellulaires exprimant un récepteur au FEEc, dans des applications médicales (comme la thérapie cellulaire) ou non médicales (comme le maintien de cellules en culture à but expérimental ou de production protéique).

### Action dans le monde animal des mammifères

La molécule présente une spécificité d'espèce. Ses isoformes sont déclinables pour une utilisation chez tous les animaux domestiques, ou non, y compris les animaux de ferme dont certaines espèces se reproduisent difficilement (chevaux de course, vache Holstein).

### Action sur les pathologies mitochondriales et contre le vieillissement

Les propriétés du FEEc comme molécule stimulant l'activité mitochondriale ou plus généralement le métabolisme énergétique peuvent également être utilisées *in vivo* dans tout type de pathologie liée à un défaut d'activité mitochondriale. A ce titre, on peut citer, de façon générale, les pathologies du vieillissement. La relation entre une dysfonction des mitochondries et les maladies neurodégénératives, par exemple, a été établie par plusieurs équipes. Aussi, le FEEc pourrait être utilisé comme médicament pour traiter les maladies neurodégénératives telles que la maladie d'Alzheimer ou la maladie de Parkinson. Il a également été montré une relation entre la longueur des télomères des chromosomes et l'activité mitochondriale de la cellule. Or le raccourcissement des télomères est lié aux processus de vieillissement. Il est donc possible qu'en stimulant l'activité mitochondriale ou énergétique de la cellule on soit à même de retarder les effets du vieillissement.

Les maladies mitochondriales présentent un tableau varié mais associent fréquemment des manifestations oculaires à type de rétinites pigmentaires ou d'ophtalmoplégie. Pour ces dernières, une administration topique du FEEc dans l'oeil, par exemple dans un collyre, pourrait améliorer les symptômes liés à l'insuffisance mitochondriale ou énergétique. Un autre objet de la description est donc un collyre comprenant un peptide cyclique comprenant le tripeptide capable de former un site de liaison de la fertiline béta à l'intégrine de l'ovocyte. Un tel collyre peut comprendre, outre le FEEc, un autre agent tel qu'un agent épaississant, un agent antiseptique, un antibiotique ou tout autre composé utilisable pour ce genre de produit. Les milieux décrits dans la demande WO 2005/051799 A2 sont bien entendu exclus de la définition du terme "collyre" au sens de la présente description.

### Action en cosmétique

La présente description porte également sur l'utilisation du FEEc, dans une composition cosmétique ou thérapeutique destinée à une application topique. A titre d'exemples, on peut citer l'utilisation du FEEc pour stimuler les fibroblastes pour la production de collagène, ou pour stimuler les follicules pileux pour favoriser la croissance des cheveux, par exemple pour prévenir ou ralentir l'alopécie. La présente description porte donc également sur une composition cosmétique ou dermatologique comprenant du FEEc à titre de principe actif. Par "composition cosmétique ou dermatologique", on entend ici une composition qui, outre le FEEc, comprend des ingrédients usuellement utilisés dans le domaine de la cosmétologie. La composition cosmétique ou dermatologique peut se présenter sous toute forme connue de l'homme du métier. Il peut s'agir, par exemple, d'une émulsion huile-dans-l'eau, eau-dans-l'huile, eau dans silicone, d'une émulsion multiple, d'une microémulsion, d'une nano-émulsion, d'une émulsion solide, d'un gel aqueux ou hydro-alcoolique, d'une crème, d'un lait, d'une lotion, d'une pommade, d'une huile, d'un baume, d'un onguent, d'un masque, d'une poudre, d'un support imbibé, par exemple un patch transdermique, d'une lotion aqueuse ou hydroalcoolique et/ou une cire, d'un produit de maquillage, par exemple un fond de teint, d'un shampooing, d'un après-shampooing, d'un masque, d'un sérum pour application topique, d'une lotion capillaire. Les milieux décrits dans la demande WO 2005/051799 A2 sont bien entendu exclus de la définition des compositions cosmétiques ou dermatologiques au sens de la présente description.

La composition cosmétique ou dermatologique peut être, par exemple une composition pour le soin du visage, du corps, des cheveux, par exemple des compositions pour le visage et/ou le corps et/ou les cheveux.

Les exemples suivants illustrent l'invention sans toutefois limiter son étendue.

### Légendes des figures

**Figure 1** : Variation du potentiel de membrane mitochondrial en présence du FEEc (à droite) *versus* peptide témoin "scramble" (à gauche). Noter l'augmentation du potentiel de membrane chez les spermatozoïdes exposés chez la majorité des patients.
**Figure 2** : Comptage après excitation aux UV des spermatozoïdes humains fusionnés avec des ovocytes humains dépellucidés, incubés en l'absence (A) ou en présence de FEEc à 100µM (B). Noter l'augmentation du nombre de spermatozoïdes fusionnés et la décondensation plus rapide de leur tête.
**Figure 3** **:** Proportion d'ovocytes matures à J1 de Maturation In Vitro (MIV). Représentation schématique des proportions d'ovocytes humains en métaphase II (MII) à partir du stade VG après MIV dans le milieu témoin ou supplémenté en FEEc à 100µM.^{∗}p=0,02. ** p=0,003. J1=24h de MIV.
**Figure 4** **:** Proportion d'ovocytes atrétiques à J1 de Maturation In Vitro (MIV). Représentation schématique des proportions d'ovocytes humains atrétiques. Résultats observés à J1 à partir du stade VG après maturation in vitro (MIV) dans le milieu témoin ou supplémenté en FEEc à 100µM. J1=24h de MIV.
**Figure 5** : Marquage du fuseau méiotique obtenu sur un ovocyte humain en MII après MIV dans le milieu standard (24h). Marquage du fuseau par un anticorps anti-α-tubuline et des chromosomes au DAPI. Image obtenue au microscope confocale. A ; ovocyte entier. B ; agrandissement de la plaque métaphasique.
**Figure 6** : Comparaison du taux de fécondation à J1 entre les souris jeunes et âgées, en présence ou en l'absence de fertiline, qui correspond au peptide QDEc.
   Jeunes : souris B6CBAF1 de 7 semaines ; Agées : souris B6CBAF1 de 7 mois
**Figure 7** **:** Comparaison des pourcentages moyens d'embryons clivés à J2 et à J4 entre les souris jeunes et âgées, en présence ou en l'absence du peptide QDEc.
   Jeunes : souris B6CBAF1 de 7 semaines (n=108 ovocytes, 56 témoins, 52 QDEc) ; Agées : souris B6CBAF1 de 7 mois (n=128 ovocytes, 65 témoins, 63 QDEc),*; p=0,02. **p=0,008. ***p=0,01.
**Figure 8** : Comparaison des pourcentages moyens d'atrésie (ATR) à J2 entre les souris jeunes et âgées, en présence ou en l'absence du peptide QDEc.
   Jeunes : souris B6CBAF1 de 7 semaines (n=108 ovocytes, 56 témoins, 52 QDEc) ; Agées : souris B6CBAF1 de 7 mois (n=128 ovocytes, 65 témoins, 63 QDEc).
**Figure 9** : Représentation schématique de la méthodologie de l'étude clinique réalisée dans l'exemple 5.
**Figure 10** : Résultats préliminaires des critères de jugement principal et secondaire dans le cadre de l'étude clinique. Taux de grossesse par transfert d'embryon frais ou congelé (groupe témoin, n=17 transferts/groupe FEEc, n=13 transferts). Pourcentage de « top embryon » (groupe témoin, n=75 embryons clivés/groupe FEEc, n=72). Taux de fécondation (groupe témoin, n=259 Mil/groupe FEEc, n=246 MII)
**Figure 11** : Taux de grossesse obtenus dans le cadre de l'étude clinique.
**Figure 12** : Amélioration du taux de maturation des ovocytes bloqués en VG après incubation en présence du FEEc. Cette étude a été réalisée en considérant un ovocyte par femme et en incubant celui-ci en présence du FEEc ou du peptide contrôle.
**Figure 13** : Effets de FEEc sur la maturation des ovocytes humains bloqués en VG par tranche d'âge.
**Figure 14** : Stimulation du développement embryonnaire pré-implantatoire chez la souris jeune par le QDEc. (*P<0,00532; **P< 0,00374; ***P< 0,00913; ****P<0,068; ( ) nombre d'embryons)

### Matériels et Méthodes

Les exemples expérimentaux présents ci-dessous ont été obtenus en utilisant les matériels et méthodes suivants :

### Observation des paramètres du mouvement spermatique

Les spermes étudiés ont chacun été divisés en 2 aliquotes dont l'une a été incubée avec le FEEc et l'autre avec un peptide « scramble » contenant les mêmes acides aminés mais dans un ordre aléatoire. Les inventeurs ont incubé pendant 3h à 37°C, des spermatozoïdes de l'espèce humaine en présence de 100 µM du peptide FEEc ou du peptide scramble puis ont observé les paramètres du mouvement spermatique selon une analyse automatisée *(Computed Assisted Sperm Analysis,* CASA).

Les paramètres spermatiques testés sont les suivants : la VAP lissée, la VSL, la VCL et l'ALH. Ils correspondent respectivement à la vitesse selon la trajectoire moyenne, la vitesse en ligne droite *(Straight Line Velocity),* la vitesse curviligne et le déplacement latéral de la tête. L'étude a montré une augmentation significative du pourcentage de spermatozoïdes hyperactivés (selon les critères de Mortimer et al.). Cela est de nature à expliquer l'augmentation des taux de fécondation observé en présence du peptide.

### Mesure du potentiel de membrane mitochondrial

Les inventeurs ont incubé pendant 3h à 37°C, des spermatozoïdes de l'espèce humaine en présence soit du peptide FEEc, soit du peptide "scramble" qui comprend les mêmes acides aminés dans un ordre aléatoire et constitue ainsi le groupe témoin. Après lavage, les spermatozoïdes sont marqués grâce à un colorant lipophile fluorescent, le DIOC6.

Le potentiel de membrane mitochondrial (gradient protonique au niveau de la membrane interne de la mitochondrie) a ensuite été mesuré par cytométrie en flux. Il se trouve augmenté dans les spermatozoïdes après exposition au FEEc.

### Mesure de l'index de fécondation

Des ovocytes humains dépellucidés ont été incubés avec des spermatozoïdes humains en l'absence ou en présence de FEEc à 100µM. Les spermatozoïdes fusionnés ont été décomptés après excitation UV. Les spermatozoïdes ont été considérés fusionnés lorsque le noyau était marqué au HOECHST 33342. De plus, les têtes des spermatozoïdes ayant pénétré l'ovocyte en présence du peptide FEEc sont non seulement plus nombreuses mais présentent également un aspect flouté témoin de la décondensation de leur tête spermatique. Cette décondensation est une des premières étapes de l'activation ovocytaire après la pénétration du spermatozoïde. Nous pouvons donc en conclure que le FEEc non seulement améliore la fécondance du spermatozoïde mais qu'il active également l'ovocyte fécondé.

### Collecte des ovocytes humains en vue de la Maturation In Vitro (MIV)

Des ovocytes humains immatures donnés à la recherche ont été collectés au sein du laboratoire de Fécondation In Vitro (FIV) du Centre d'Assistance Médicale à la Procréation (AMP) de l'hôpital Cochin (Paris, France). Deux heures après la ponction ovocytaire, les ovocytes destinés à être microinjectés ont été décoronisés par la hyaluronidase (ORIGIO, Limonest, France). Après observation au microscope inversé (Hoffman), les ovocytes immatures au stade vésicule germinative (VG) ont été retenus pour la suite des expériences.

### Maturation In Vitro (MIV) des ovocytes humains immatures

Les ovocytes humains immatures au stade VG ont été randomisés, soit dans le milieu de culture témoin (Global, JCD, La Mulatière, France) (n=203) soit dans le même milieu supplémenté avec 100 µM de FEEc (n=193). Les ovocytes humains immatures ont été classés en deux groupes : ceux appartenant aux femmes âgées de moins de 37 ans et ceux appartenant à celles âgées de 37 ans et plus. Le jour de la ponction folliculaire, les deux groupes d'ovocytes au stade VG ont été incubés dans des gouttes de 20µl recouvertes d'huile, et maintenus à 37°C sous 5% de CO₂ pour être observés au microscope inversé (Hoffman) à J1 (24h d'incubation) et J2 (48h d'incubation). Les ovocytes étaient classés en métaphase II (1er globule polaire dans l'espace périvitellin), vésicule germinative (VG), métaphase I (rupture de la vésicule germinative sans expulsion du globule polaire) ou atrétiques.

### Collecte des ovocytes murins en vue de la Maturation In Vitro (MIV)

Des femelles B6CBAF1 (âgées entre 5 et 8 semaines) fournies par le laboratoire Charles River (L'Arbresie, France) ont été stimulées par injection de PMSG *(Prégnant Mare Serum Gonadotrophin)* à 10UI (SIGMA-ALDRICH, Saint-Quentin Fallavier, France) sans déclenchement de l'ovulation. Les ovocytes immatures ont été recueillis dans les ovaires 48h après cette dernière injection, puis dénudés de leurs cumulus grâce à la hyaluronidase et lavés à trois reprises dans du milieu de culture M2. Seuls les ovocytes classés en VG ont été retenus pour la suite des expériences.

### Maturation In Vitro (MIV) des ovocytes de souris immatures

Les ovocytes de souris ont été incubés de manière randomisée entre le milieu standard d'une part et le milieu supplémenté en QDEc 100 µM d'autre part. Les boîtes de culture ont été préparées la veille et incubées à 37°C sous 5% de CO₂. Les ovocytes ont été observés à J0 (8h post-sacrifice) et J1 (24h).

### Immunofluorescence

Les ovocytes humains issus de MIV ont été fixés dans le paraformaldehyde (PFA) 2% pendant 1h à température ambiante puis lavés dans le PBS contenant 0,5% de BSA. La perméabilisation a été assurée par incubation des ovocytes dans une solution contenant 0,5% de BSA, 0,1% de Triton X-100, 0,05% de Tween-20 et 5% de sérum normal de chèvre. Puis les ovocytes ont été lavés dans le PBS-BSA 0,5% avant d'être incubés toute la nuit dans une dilution au 1/200 d'anticorps anti-α tubuline humaine (SIGMA-ALDRICH) dans du PBS contenant 0,5% de BSA. Les ovocytes ont ensuite été incubés 1h en présence de l'anticorps secondaire de type IgG conjugué à l'Alexa Fluor (LIFE TECHNOLOGIES, Alfortville, France). Après une étape de lavage, les ovocytes ont été incubés 10 min dans du DAPI (dilué au 1/1000) avant d'être montés sur lame et observés en microscopie confocale dans l'obscurité. Pour l'analyse du fuseau, les ovocytes avec des fibres de microtubules distincts et bien organisés associés à un alignement parfait des chromosomes au niveau de la plaque métaphasique sont identifiés comme normaux.

### Stimulation et accouplements des souris

Des femelles B6CBAF1 "jeunes" âgées de 7 semaines et "âgées" de 7 mois ont été accouplées avec des mâles C57N après superovulation, cette dernière consistant à injecter la PMSG à 10UI (SIGMA-ALDRICH) suivi du déclenchement de l'ovulation par administration d'hCG (Human Chorionic Gonadotrophin) à 10 UI (SIGMA-ALDRICH), 46-48h après.

Le lendemain de l'accouplement, les souris présentant un bouchon vaginal ont été sacrifiées. Les ovocytes ont été recueillis dans les oviductes 15-16h après l'injection d'hCG et le taux de fécondation a été évalué par la présence du second globule polaire dans l'espace périvitellin.

### Incubation des ovocytes fécondés de souris

Les ovocytes de souris fécondés après accouplement et recueillis ont été randomisés en 4 groupes (jeunes «exposées au peptide QDE cyclique (QDEc), jeunes contrôles, âgées exposées au QDEc, âgées contrôles) et disposés dans des gouttes de milieu de culture (KSOM) de 20µL pour les témoins et supplémentés avec 100µM de QDEc pour les exposés. L'exposition a duré de J1 à J4 après l'accouplement *in vivo* (J0). Le QDEc est équivalent au FEEc humain. L'incubation des boites de culture est réalisée à 37°C sous 5% de CO₂ et elles sont recouvertes d'huile minérale.

Les ovocytes ont été observés tous les jours à la loupe binoculaire pour apprécier les signes de développement embryonnaire. La cinétique de développement normale correspond au minimum, à J2, à un embryon clivé à 2 cellules et à J5 un embryon au stade morula ou blastocyste.

### Etude prospective randomisée en FIV humaine

Un essai clinique a débuté au sein du laboratoire de FIV du Centre d'AMP de Cochin, le 08/09/2014, sur 66 couples, la moyenne d'âge étant de 34,3± 4,2 ans pour les femmes et 37,0± 5,2 pour leurs partenaires. Il s'agit d'une étude prospective, monocentrique randomisée, des Fécondation In Vitro (FIV) réalisées en présence ou en l'absence de FEEc. Les ovocytes, récupérés dans leurs cumulus, ont été répartis en deux groupes alternativement dans l'un puis dans l'autre en fonction de leur ordre de récupération. Lorsque tous les ovocytes ont été récupérés, un technicien n'ayant pas participé au recueil des cumulus déterminait par randomisation lequel des deux groupes était inséminé en présence de FEEc et lequel servait de témoin. Une partie des ovocytes est incubée dans le milieu de culture standard (Global, JCD), l'autre partie dans ce même milieu supplémenté en FEEc 100µM.

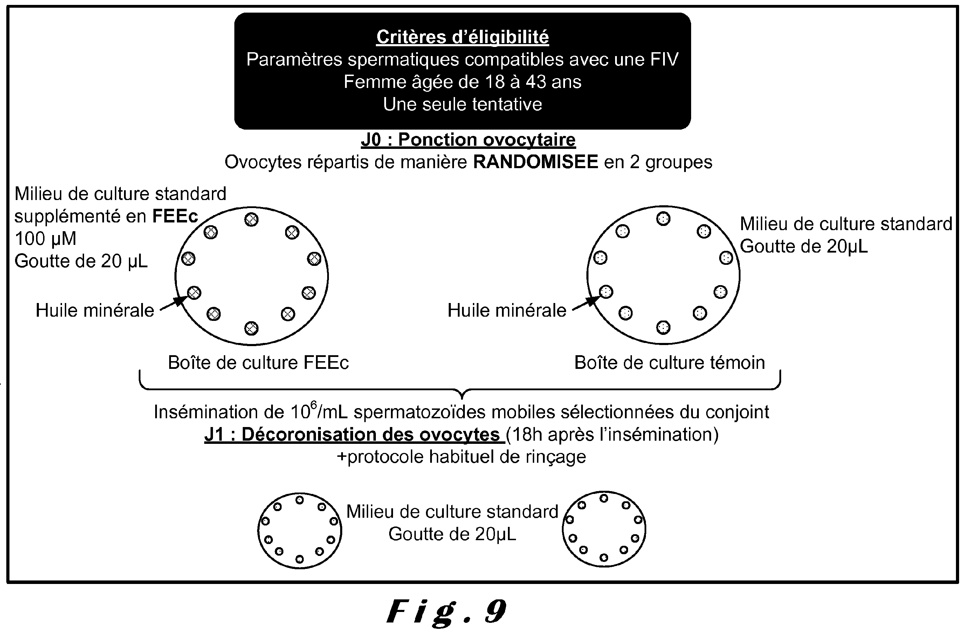

La méthodologie de cette étude, schématisée à la figure 9, est présentée ci-dessous. Des ovocytes humains provenant de femmes âgées de 18 à 43 ans ont été récupérés par ponction des ovaires après stimulation hormonale par les gynécologues. Ils ont été répartis de manière randomisée en 2 groupes : les ovocytes incubés en présence d'un milieu de culture standard supplémenté en FEEc à 100 µM ou d'un milieu de culture standard (Global, JCD) puis placés dans un incubateur à 37° sous une atmosphère de 5% de CO₂. Les spermatozoïdes du conjoint ont été récupérés au laboratoire et les plus mobiles, sélectionnées selon une préparation standard des centres d'AMP. La FIV consiste en la mise en présence des spermatozoïdes et des ovocytes dans le milieu d'insémination à raison d'une concentration de 10⁵ spermatozoïdes sélectionnés /ml dans des gouttes de 20 microlitres sous huile. L'insémination a lieu dans un incubateur à 37°C sous 5% de CO₂ pendant 18 heures. 18h après l'insémination (J1), la décoronisation des ovocytes est effectuée. Les ovocytes fécondés sont lavés et transférés dans une autre goutte de milieu et mis en culture pour 24h supplémentaires. Lors du transfert in utero, ils sont lavés trois fois, puis mis dans un milieu de transfert et placés dans la cavité utérine. Les embryons sont transférés selon leur qualité apparente sans se soucier du groupe d'origine. Un, ou plusieurs embryons sont transférés en fonction de l'âge, de l'indication de la FIV, du rang de la tentative et de la qualité des embryons obtenus, et avec l'accord des couples. Certains embryons peuvent être mis en culture prolongée sur 5 jours (au stade blastocyste) soit d'emblée, soit après transfert d'embryons à J2.

Le critère d'évaluation principal est le taux de grossesse clinique par transfert d'embryon frais ou congelé et le taux de fausse couche en tenant compte des 3 groupes : transferts homogènes (témoins et traités) et mixtes (mélange des deux).

Les critères secondaires sont :
- Les taux de fécondation, i.e., le rapport du nombre de zygotes ayant deux pronucleus dans le cytoplasme, 18 heures après insémination rapporté au nombre d'ovocytes en métaphase 2 dans la cohorte.
- Le pourcentage d'embryon de bonne qualité *i.e.,* des embryons dont la séquence de clivage correspond à la séquence idéale soit : 4 à 5 cellules à J2 et 8 à 9 cellules à J3 et dont la fragmentation des blastomères est de type A (lorsque le volume occupé par les fragments est inférieur à 10% du volume embryonnaire) ou B (lorsque le volume occupé par les fragments est compris entre 10% et 30% du volume total de l'embryon). Ainsi ces embryons dit « Top » correspondent au rapport du nombre d'embryons de chaque type rapporté au nombre d'embryons total pour chaque groupe.

Ce protocole a reçu l'avis favorable du Comité de Protection des Personnes (CPP) Ouest VI le 13/12/2012. L'étude a obtenue l'autorisation de l'Agence de la Biomédecine le 08/07/2013. La FIV est effectuée dans le strict respect des Bonnes Pratiques Cliniques. Chaque couple acceptant de participer à l'étude a signé un consentement libre et éclairé.

### Analyse statistique

Les variables quantitatives ont été étudiées par leurs effectifs, moyenne et écart-type. Les données ont été comparées entre le groupe exposé et non exposé à l'aide d'un test approprié (test de Student ou test de Wilcoxon) pour les variables quantitatives. Les comparaisons de pourcentages ont été effectuées grâce à un test de Chi-2 (χ2) ou un test exact de Fisher. Les différences entre les données comparées ont été considérées comme statistiquement significatives lorsque la valeur de p (seuil de significativité) était inférieure à 0,05.

### Exemple 1 : Amélioration des paramètres du mouvement des spermatozoïdes et du pourcentage de spermatozoïdes hyperactivés chez l'Homme

Des expériences préliminaires ont montré que dans un test de survie sur 18 heures de spermes incubés en présence du peptide FEE, la survie est significativement améliorée dans le groupe traité par le FEE à la concentration de 100µM comparé au témoin.

### Exemple 1a: Analyse automatisée des paramètres du mouvement spermatique après incubation en présence de FEEc et d'un peptide scramble pour témoin.

Les résultats présentés dans le tableau 1 ci-dessous montrent une augmentation de la VAP lissée (p=0,008), de la VSL (p=0.048), de la VCL (p<0,0001), de l'ALH (p=0,002), résultant en une augmentation de 29% des spermatozoïdes hyperactivés (p=0,009) par rapport au groupe témoin. Cette amélioration du pourcentage des spermatozoïdes hyperactivés explique l'amélioration de leur capacité fusiogène et l'augmentation des taux de fécondation enregistrés chez la souris sur des complexes cumulo ovocytaire intacts.

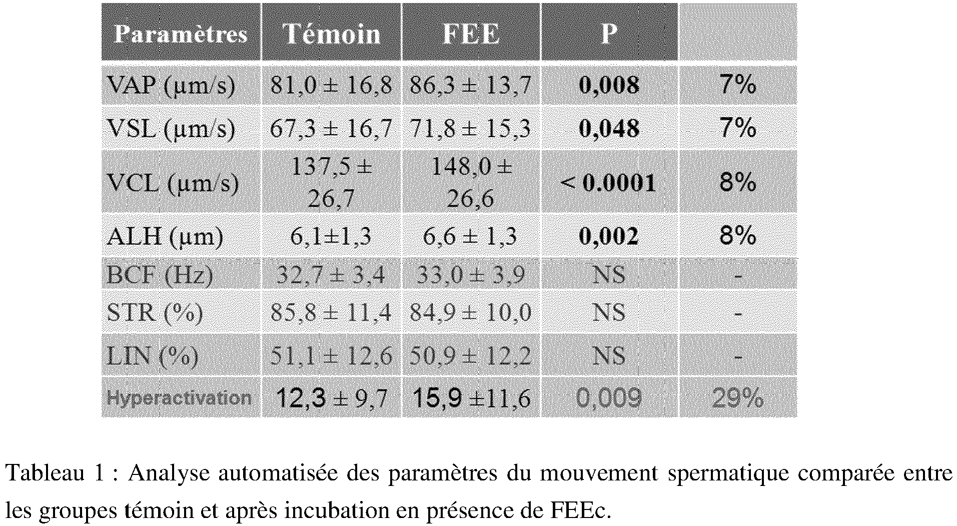

### Exemple 1b : Mesure du potentiel de membrane mitochondrial

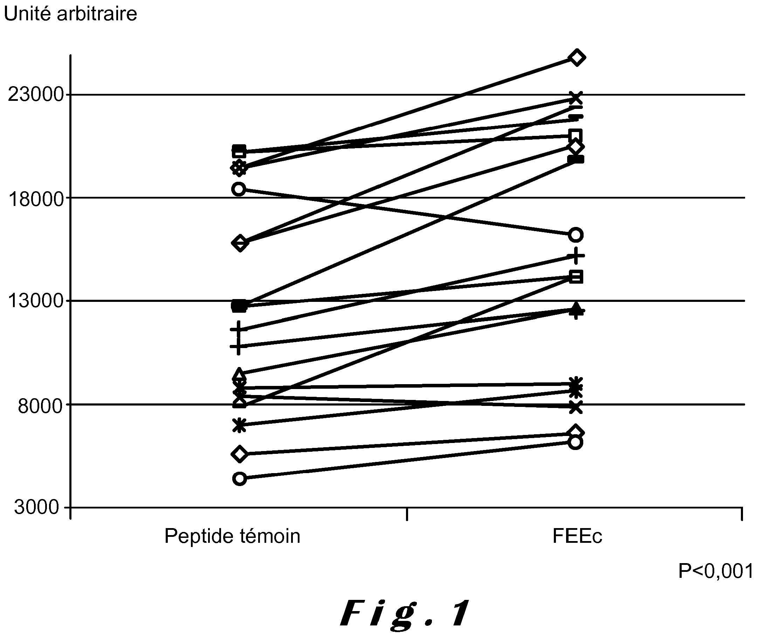

Le potentiel de membrane mitochondrial s'avère être augmenté de 21% en présence du peptide FEEc par rapport au peptide « scramble » (p<0.001) (Figure 1).

Le FEEc améliore donc les paramètres du mouvement spermatique par augmentation du potentiel de membrane mitochondrial spermatique.

### Exemple 1c : Etude de l'index de fécondation

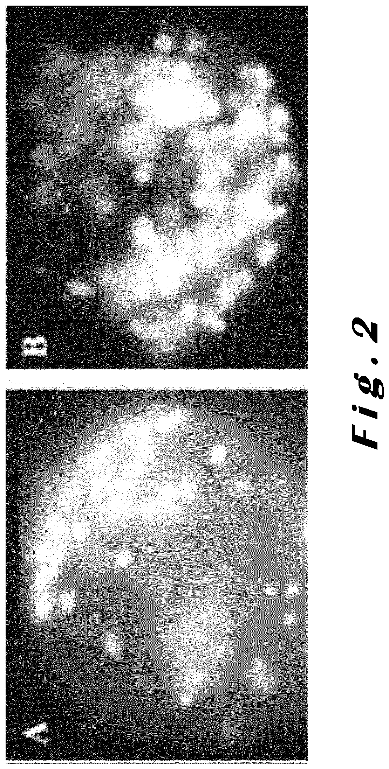

Les résultats présentés à la figure 2 montrent que, non seulement un plus grand nombre de spermatozoïdes est fusionné avec les ovocytes dépellucidés en présence du peptide FEEc, mais ces derniers sont également décondensés contrairement à ceux du groupe témoin.

Sur les ovocytes témoins, on dénombre une moyenne de 19,0 ± 4,6 spermatozoïdes dans le cytoplasme alors qu'une augmentation avec 36,9 ± 11,7 spermatozoïdes fusionnés par ovocytes, est rapportée après incubation avec le FEEc à 100 µM (p<0,001). Ce phénomène suggère une amélioration de la fécondance des spermatozoïdes et une activation ovocytaire médiée par le peptide FEEc.

Les paramètres du mouvement des spermes de 37 patients ont été analysés en présence ou en absence de FEEc (Incubation de 3 heures). Il y a une augmentation significative du pourcentage de spermatozoïdes hyperactivés selon les critères de Mortimer, ce qui explique l'augmentation de leur fécondance (voir Tableau 1 ci-dessus).

### Exemple 2 : Amélioration des pourcentages de maturation in vitro des ovocytes humains immatures

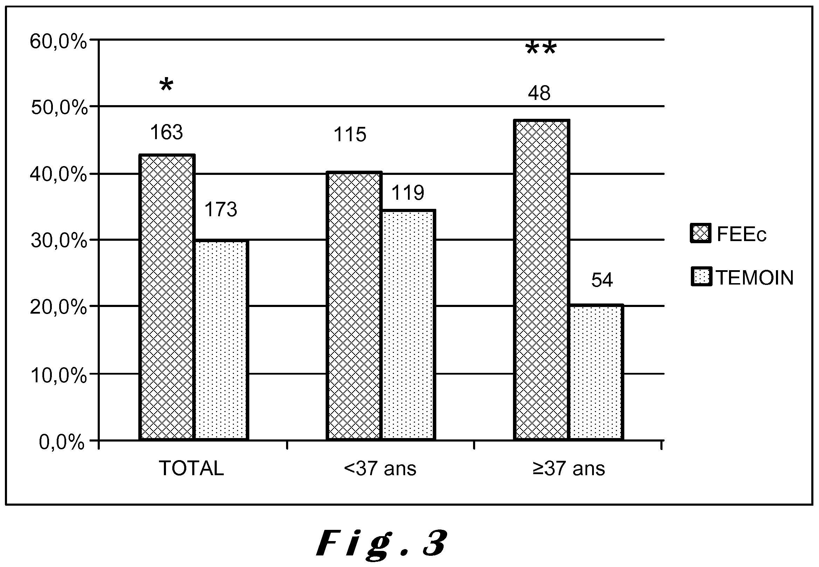

Une augmentation significative de la maturation ovocytaire sous FEEc a été mise en évidence à J1, pour l'ensemble des ovocytes humains testés. Les résultats obtenus sont les suivants: 42,3% (69/163) d'ovocytes en métaphase II (MII) sous FEEc *versus* 30,0% (52/173) dans le groupe témoin, p=0,02 (Figure 3). Cette augmentation de maturation est encore plus marquée pour les ovocytes issus de femmes âgées de 37 ans et plus dès J1. Les résultats obtenus sont les suivants: 47,9% (23/48) d'ovocytes en MII sous FEEc *versus* 20,4% (11/54) dans le groupe témoin, (p=0,003).

Bien que faible pour les ovocytes issus de femmes dont l'âge est inférieur à 37 ans, l'amélioration de la MIV ovocytaire chez l'humain est très significative pour les ovocytes de femmes plus âgées puisque le même taux de maturation que pour les ovocytes des femmes jeunes a été obtenu (47,9% des ovocytes en métaphase II en présence de FEEc *versus* 20,4% dans le groupe témoin, p=0,003).

Les résultats obtenus avec les 336 ovocytes humains au stade VG, incubés de manière randomisée en présence ou en l'absence du peptide FEEc, montrent que la présence du FEEc améliore le taux de maturation des ovocytes humains.

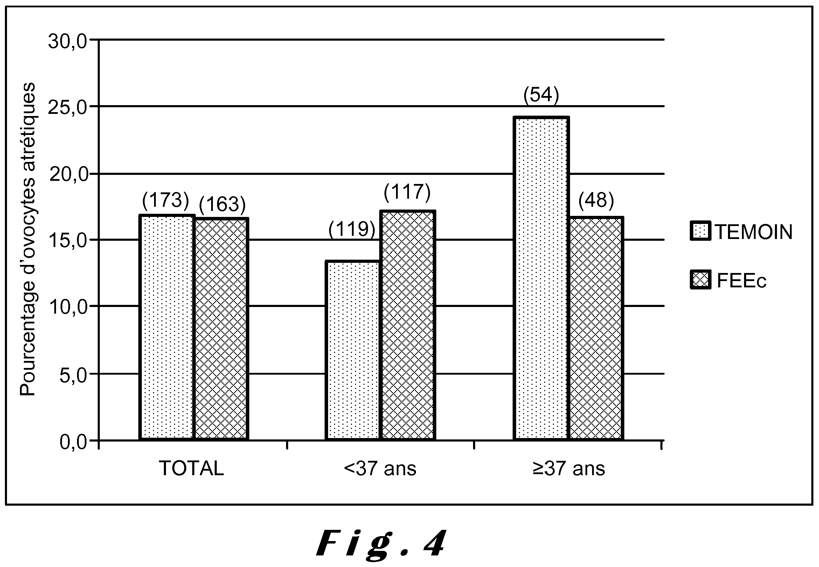

Aucune différence significative entre les taux d'atrésie ovocytaire parmi les 2 groupes n'a été détectée (16,5% en présence du peptide *versus* 16,8% pour le groupe témoin) (Figure 4). Dans l'échantillon de femmes âgées de 37 ans et plus, on note une tendance non significative à une diminution du taux d'atrésie en présence du peptide FEEc (16,7%, 8/48) par rapport au milieu témoin (24,1%, 13/54) p>0,05.

L'étude a été poursuivie et des résultats complémentaires ont été obtenus. Ces résultats confirment les résultats décrits ci-dessus et mettent en évidence d'autres effets du peptide FEEc, comme décrits ci-après.

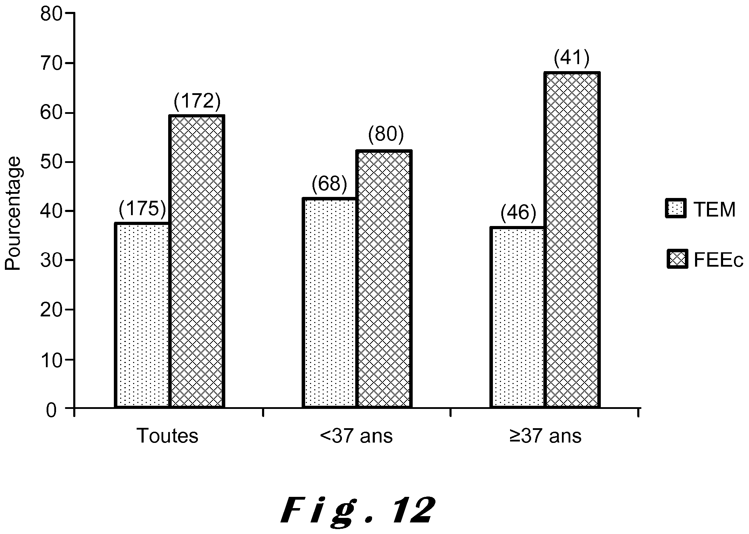

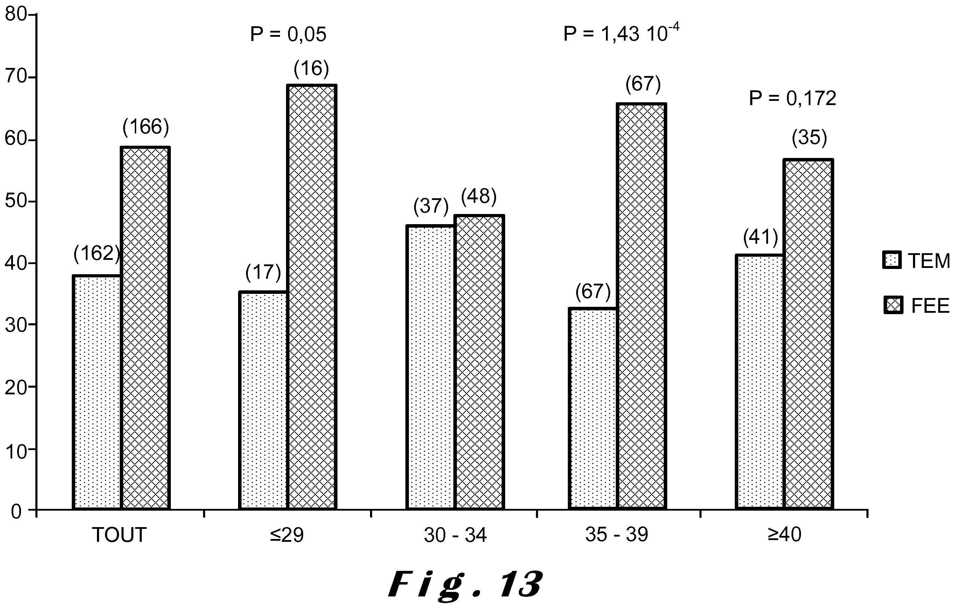

Au total 600 ovocytes ont été mis en maturation *in vitro.* Pour l'analyse, seulement un ovocyte par femme a été inclus dans l'étude afin que les événements soient tous indépendants. En présence de la Fertiline dans le milieu, le taux de maturation est passé de 38,3% à 59,0% (P<1.6 10⁻⁴) (Figure 12). Quand l'analyse est faite en fonction de l'âge de la femme dont proviennent les ovocytes en VG, on voit que l'amélioration de la maturation est relativement modeste pour les femmes de moins de 37 ans (42,6% à 51,8%, P<0,2), mais qu'elle est beaucoup plus important chez les ovocytes provenant de femmes âgées de 37 ans et plus (35.1% à 65.9% P<3.91 10⁻⁵). La Fertiline est donc à même de stimuler la maturation *in vitro* de l'ovocyte décoronisé et l'expulsion du premier globule polaire. Elle le fait apparemment d'autant mieux que le déficit énergétique de l'ovocyte lié à l'âge est plus important. Cette étude a également permis de mettre en évidence une augmentation du taux de maturation plus particulièrement les ovocytes en VG des femmes de plus de 37 ans et de moins de 30 ans (Figure 13).

### Exemple 3 : Organisation du fuseau méiotique des ovocytes humains maturés in vitro

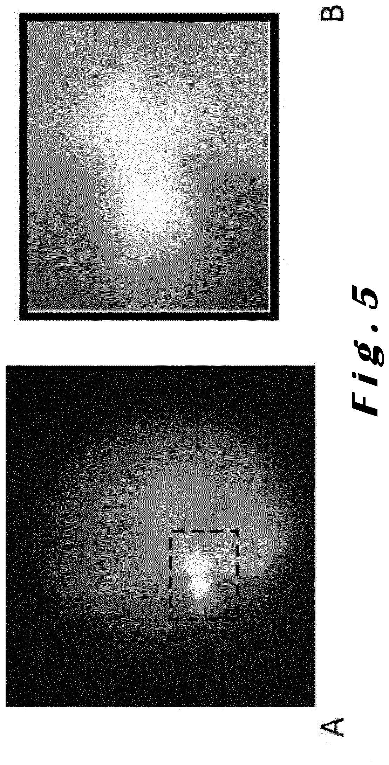

La figure 5 montre l'organisation incomplète et aberrante de l'alignement des chromosomes sur la plaque métaphasique avec des chromosomes non alignés. L'image est obtenue à partir d'un ovocyte humain en MII après 24h de MIV en présence du milieu contrôle.

### Exemple 4 : Amélioration du taux de fécondation et du développement embryonnaire précoce chez la souris

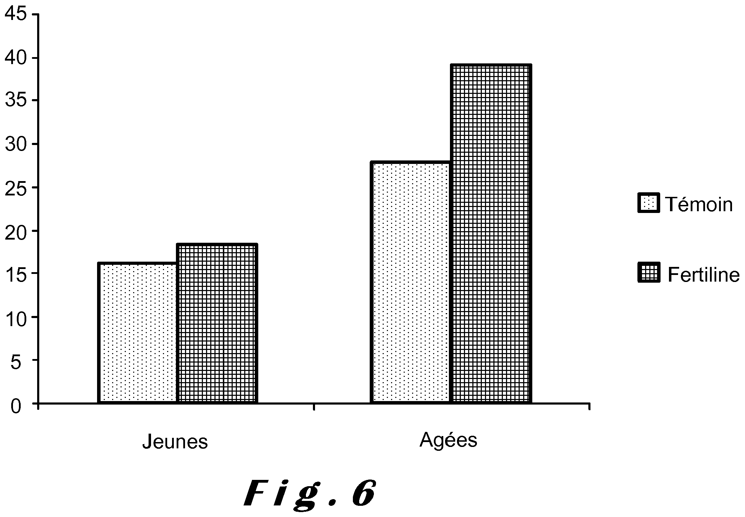

A J1, le taux de fécondation reste inchangé chez les souris jeunes, tandis qu'il passe de 39% à 51% chez les souris âgées (p<0.03) (Figure 6).

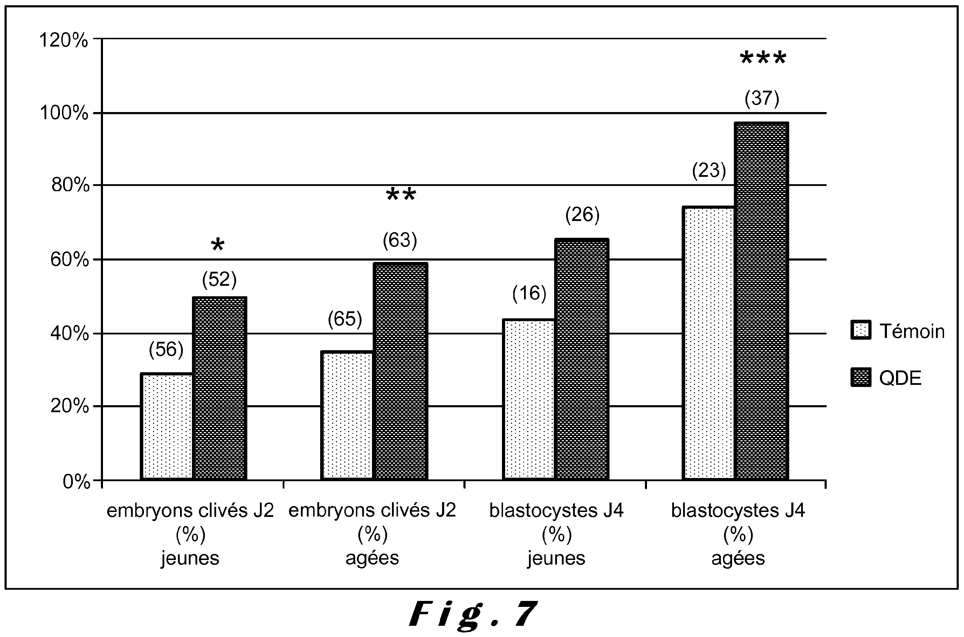

A J2 parmi les souris jeunes, 50,0% (26/52) des ovocytes sont clivés dans le groupe QDEc contre 32,4 % (18/56) dans le groupe témoin (P=0,02) (Figure 7). Concernant les souris âgées, il y a significativement plus d'embryons clivés à J2 en présence de QDEc (58,7%, 37/63) par rapport au témoin (35,4%, 23/65), p=0,008. A J4, la proportion d'embryons clivés issues de souris jeunes atteignant le stade morula ou blastocyste est de 34,6% (7/16) pour le groupe témoin contre 63,0% (17/26) pour le milieu supplémenté en QDEc, p<0,03. Cette proportion atteint 86,3 % (63/73) pour les souris âgées en présence du peptide contre 28,3% (15/53) dans le milieu témoin, p=0,001.

De manière intéressante, la blastoformation chez la souris est améliorée en présence du peptide QDEc.

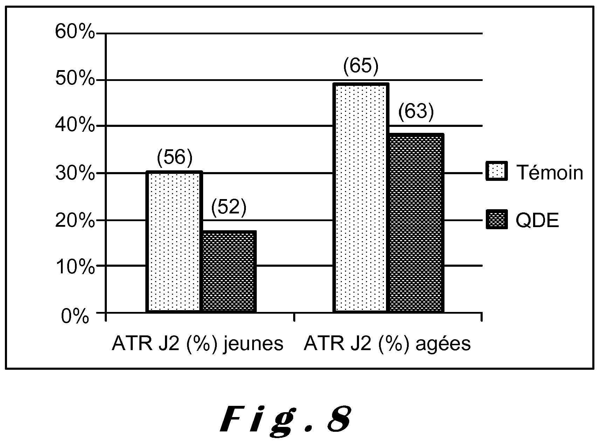

Les pourcentages d'embryons atrétiques ne sont pas significativement différents entre les 2 groupes parmi les souris jeunes ; 17,3% (9/52) en présence de QDEc et 30,4% (17/56) pour le contrôle (p=0,1). Par rapport aux souris jeunes, le pourcentage d'atrésie est plus important dans le groupe des souris âgées avec des taux similaires entre les groupes QDEc (38,1%,24/63) et témoin (49,2%, 32/65) (p=0,2) (Figure 8).

Afin d'étudier plus finement le développement embryonnaire préimplantatoire, le protocole suivant a été mis en oeuvre : Des souris ont été accouplées à J0. A J1, les ovocytes ont été récupérés par dilacération des ampoules tubaires. Ils ont alors été distribués en deux groupes de façon randomisée et mis en culture avec ou sans QDEc, avant fécondation.

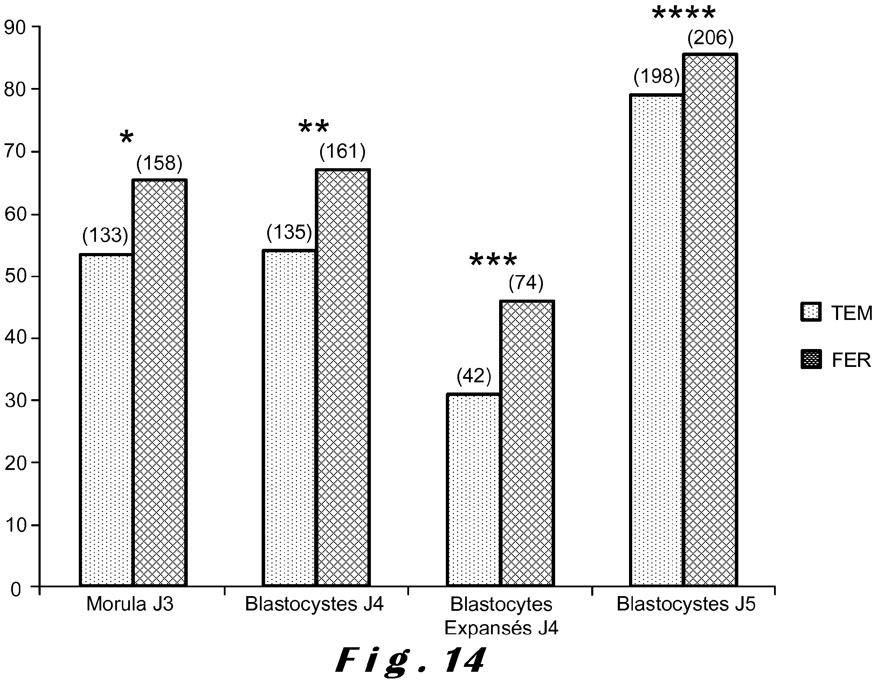

Les résultats montrent une augmentation des morulas à J3, des blastocystes à J4 et parmi eux une augmentation des blastocystes expansés lorsque l'ovocyte a été incubé en présence de QDEc (31,1% vs 45,9% P<0,009) (Figure 14). De plus, après transfert de ces blastocystes chez des femelles pseudo gestantes, il y a une augmentation significative du nombre de progénitures obtenues par embryons transférés, surtout pour les embryons provenant de souris jeunes (Tableau 2 ci-dessous).

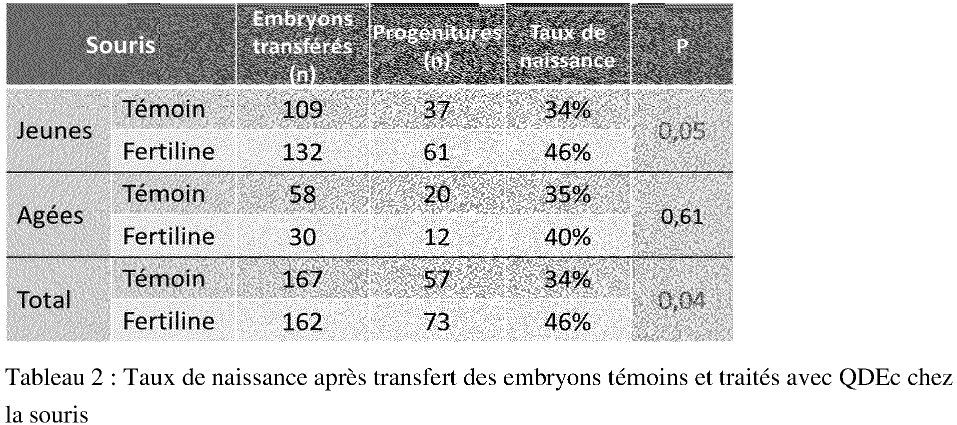

### Exemple 5 : Augmentation des taux de grossesse clinique en Fécondation in vitro

Les résultats présentés ci-dessous ont été obtenus dans le cadre de l'étude « Fertiline ».

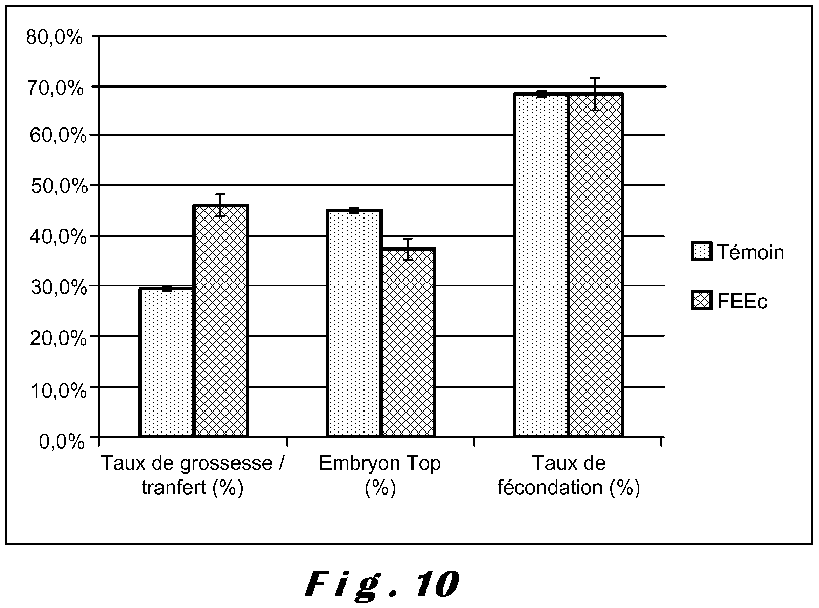

Dans un premier temps, 56 couples ont été inclus. La moyenne d'âge est de 33,9+/-4,1 ans parmi les femmes et 36,7+/-5,3 pour les partenaires. Aucune différence significative n'a été relevée, ni entre les taux de fécondation, ni entre le pourcentage d'embryons de top qualité parmi les 2 groupes FEEc versus témoin. Une tendance non significative à l'augmentation des taux de grossesse par transfert est relevée en présence du peptide par rapport au contrôle et au groupe mixte, 46,1% (6/13) contre 29,4% (5/17) et 40% (2/5), respectivement (Figure 10 et Tableau 2 ci-dessous). Une fausse couche a été rapportée après transfert d'un embryon issu du groupe témoin. Aucun effet indésirable n'a été rapporté à ce jour.

A ce jour 66 couples ont été inclus dans l'étude. 54 transferts ont été effectués, 26 avec des embryons témoins, 22 avec des embryons du groupe FEE, 6 avec des embryons des deux groupes (transferts mixtes). Le taux de grossesse cumulée a été respectivement de 34,6%, 45,5% et 33,3% dans les 3 groupes. Le taux de fausses couches spontanées était respectivement de 33,3 %, 10% et 0%. Le taux de grossesses cliniques est donc respectivement de 23%, 41% et 33,3%. Fait essentiel quand le taux de grossesse clinique est rapporté à l'âge des patientes, on s'aperçoit que chez les patientes jeunes, le taux de grossesse clinique évolutive (c'est-à-dire allant jusqu'à terme) est significativement augmenté de 20% à 57,1% (P<0,03).

**Tableau 3 : Taux de grossesse par transfert (pour 66 patients)**

| Transfert d'embryons | Témoin | FEEc | Mixte | Total |
|---|---|---|---|---|
| Patient inclus | | | | 66 |
| Transferts différés | | | | 21 |
| Transférés cumulés | 26 | 22 | 6 | 54 |
| Taux de grossesse n (%) | 9 (34,6%) | 10 (45,5%) | 2 (33,3%) | 38,9% |
| Fausses couches n (%) | 3 (33,3%) | 1 (10,0%) | 0 | 4 (19%) |

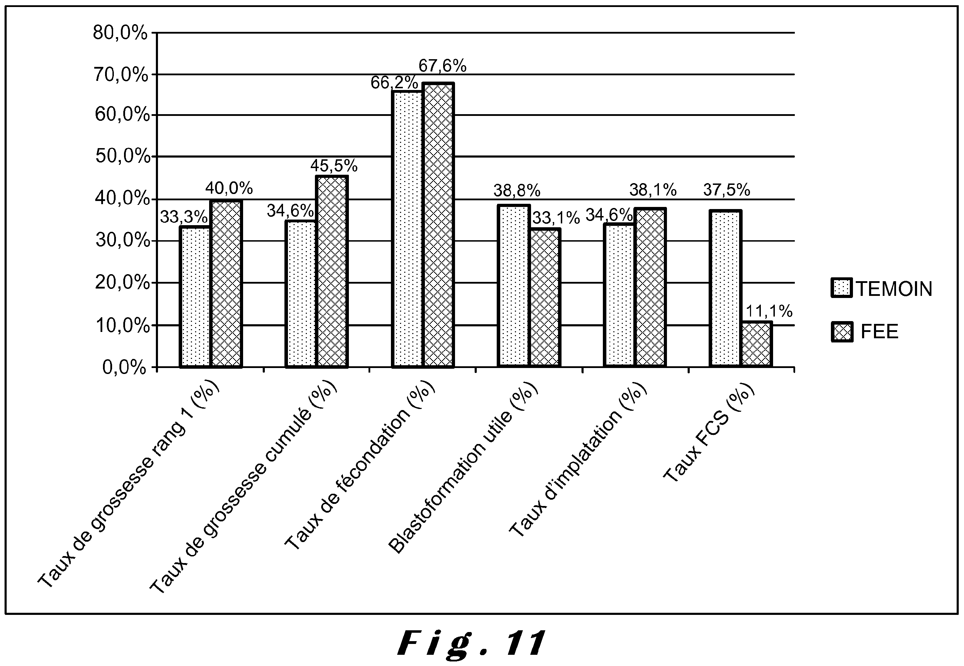

Les résultats montrent également une augmentation de 21% de grossesses cliniques après transfert d'un embryon issu du groupe FEEc 40,0% (8/20) contre 33,3% (8/24) dans le groupe témoin (p>0,05). Les taux de fécondation sont de 66,2% parmi les témoins contre 67,6% dans le groupe exposé au FEEc (p>0,05). Le taux de fausse couche spontanée précoce atteint 37,5% (3/8) dans le groupe témoin contre 11,1%. (1/9) lorsque l'embryon a été exposé au FEEc (p>0,05) (Figure 11).

Pour les femmes âgées de moins de 37 ans, après exposition au FEEc, les taux de fécondation sont de 70,9% contre 68,3% dans le groupe témoin. Dans le cas où l'ovocyte a été exposé au FEEc, les taux de grossesse atteignent 57,1% contre 20,0% dans le groupe témoin (Tableau 4 ci-dessous).

**Tableau 4 : Taux de fécondation et taux de grossesse selon l'âge de la partenaire (≥37 ou < 37 ans) * P<0,03)**

| Age femme (année) | Nombre couple (n) | Taux fécondation Témoin (%) | Taux fécondation FEEc (%) | Taux de grossesse Témoin (%) | Taux de grossesse FEEc (%) |
|---|---|---|---|---|---|
| 26-36 | 47 | 68.3% | 70.9% | 20.0% | 57.1%* |
| 37-43 | 19 | 60.2% | 60.3% | 33.0% | 20.0% |

Sur les 66 premiers couples inclus dans l'étude jusqu'à ce jour, 51 transferts ont été effectués, les autres étant différés et les résultats montrent que sur ces 51 transferts :
- 21 ont été réalisés à partir d'embryons du groupe témoin ;
- 18 à partir du groupe en présence du FEEc ;
- 12 avec des embryons des deux groupes.

Le taux de grossesse est plus élevé et les fausses couches sont moins nombreuses pour le groupe en présence du FEEc par comparaison avec les autres groupes (33% de fausses couches versus 9% pour les embryons en présence de FEEc) (Tableaux 5 et 6 ci-dessous).

Les taux de grossesses pour les femmes jeunes passent de 20% à 57,1% (p<0.03), en présence de FEEc par rapport au témoin.

**Tableau 5 : Taux de grossesse par transfert (pour 66 patients)**

| Transfert d'embryons | Témoin | FEEc | Mixte | Total |
|---|---|---|---|---|
| Patient inclus | | | | 66 |
| Transferts différés | | | | 15 |
| Patients transférés | 21 | 18 | 12 | 51 |
| Taux de grossesse n (%) | 34.6% | 45.5% | 33.3% | |
| Fausses couches n (%) | 33% | 10% | 0 | |
| Taux de grossesse évolutive (%) | 23% | 41% | 33% | |

**Tableau 6 : Taux de grossesse par transfert en fonction de l'âge des femmes.**

| | Couples | Taux de fécondation | | Taux de grossesse/transfert | | |
|---|---|---|---|---|---|---|
| Age F | N | Témoin (%) | FEE (%) | Témoin (%) | FEE (%) | Fisher |
| 26-36 | 47 | 68,3% | 70,9% | 20% (4/20) | 57,1 % (8/14) | P=0,03 |
| 37-43 | 19 | 60,2% | 60,3% | 33% (2/6) | 10% (1/10) | P=0,5 |
| Total | 66 | 66,23% | 67,6% | 23 % (6/26) | 37,5% (9/24) | P=0,2 |

La poursuite de l'étude Fertiline a permis d'obtenir des résultats complémentaires. Au total 66 couples ont été inclus dans l'étude. Les résultats sont rapportés dans les tableaux ci-dessous.

Les données globales des tentatives sont rapportées dans le tableau 7. Les taux de fécondation globaux n'ont quasiment pas été modifiés. Mais le pourcentage des tentatives pour lesquelles il y a eu une paucifécondation (moins de 20% de taux de fécondation) a chuté de 37,9% à 27,3% en présence de FEEc, suggérant une meilleure fécondance des gamètes en présence de Fertiline. De même la polyspermie est du même ordre de grandeur dans le groupe Fertiline que dans le groupe témoin (4,0% vs 5,2%) montrant que le mécanisme normal du bloc à la polyspermie n'a pas été modifié.

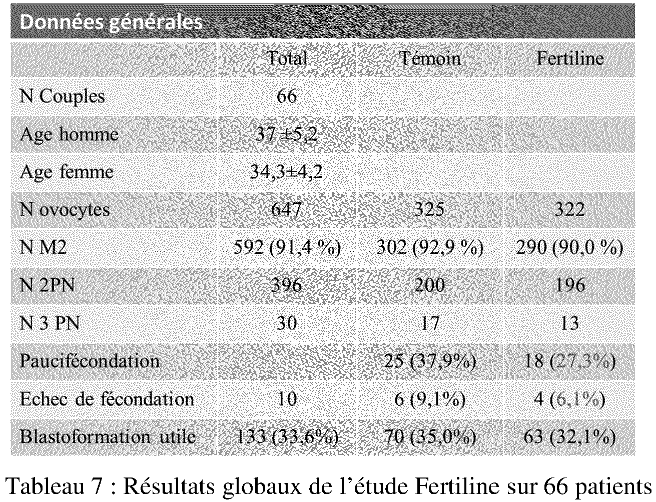

Les résultats de tous les couples ayant eu un transfert d'embryons sont rapportés dans le tableau 8. Ce tableau exclue les couples qui ont eu un échec de fécondation inexpliqué dans les deux groupes d'ovocytes avec ou sans Fertiline (n=6).

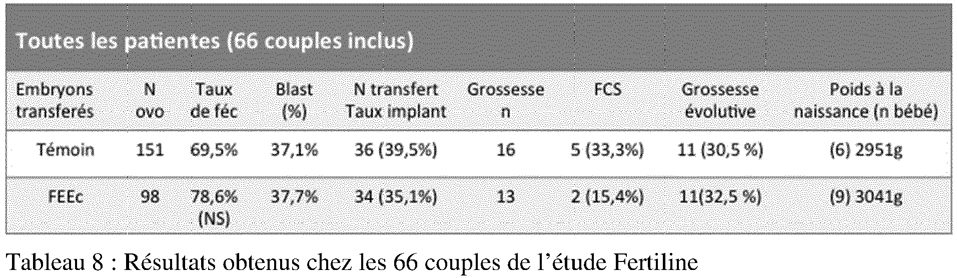

Comme le montre le tableau 8, pour les patientes ayant eu un transfert d'embryon, le taux de fécondation est passé de 69,5% à 78,6% ce qui est une amélioration de 13% mais qui n'atteint pas la significativité dans la cohorte présente. Le développement embryonnaire jusqu'au stade blastocyste n'est pas modifié, ni le taux d'implantation des embryons dans l'utérus. Par contre le taux de fausses couches est diminué de près de 50% dans le groupe Fertiline (15,4% vs 30,5%).

Les résultats des couples ayant eu un transfert d'embryons et dont la femme a moins de 37 ans sont rapportés dans le tableau 9.

Si l'on ne considère que les couples dont la femme a moins de 37 ans (n=47) et qui correspondent à plus de 70% des patientes prises en charge, on s'aperçoit que le taux de fécondation est significativement amélioré de 70,9% à 83,3% (P<0,05). Le taux des fausses couches passe de 36,3%, pour les patientes ayant reçu un embryon du groupe Témoin à 9,1% soit quatre fois moins important, pour celles ayant reçu un embryon du groupe Fertiline. De fait, le taux des grossesses évolutives donnant naissance à un enfant passe de 28,6% dans le groupe des embryons Témoins à 41,6% dans celui des embryons du groupe Fertiline.

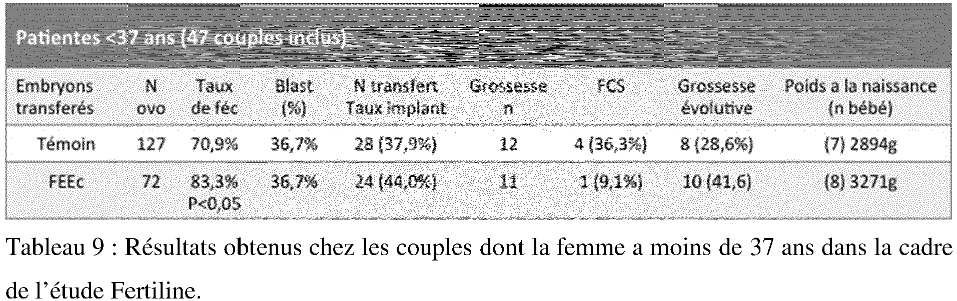

## Revendications

1. Utilisation *in vitro* d'un peptide cyclique comprenant le peptide cyclique de formule C-S-F-E-E-C (SEQ ID NO: 1) avec une liaison de cyclisation entre les deux cystéines terminales reproduisant un site de liaison de la fertiline béta à l'intégrine de l'ovocyte, pour la maturation *in vitro* d'ovocytes immatures.

2. Utilisation selon la revendication **1,** dans laquelle les ovocytes sont des ovocytes humains.

3. Utilisation selon la revendication **1** ou **2,** dans laquelle les ovocytes sont incubés pendant une durée comprise entre 1 minute et 4 jours, en particulier jusqu'à 24 heures, en présence de 1 à 100 µM de peptide.

4. Utilisation selon l'une des revendications **1** à **3,** pour améliorer la ploïdie des ovocytes.

5. Utilisation *in vitro* d'un peptide cyclique comprenant le peptide cyclique de formule C-S-F-E-E-C (SEQ ID NO: 1) avec une liaison de cyclisation entre les deux cystéines terminales reproduisant un site de liaison de la fertiline béta à l'intégrine de l'ovocyte, pour la cryopréservation des gamètes et des embryons.

## Patentansprüche

1. In-vitro-Verwendung eines zyklischen Peptids, das das zyklische Peptid der Formel C-S-F-E-E-C (SEQ ID NO: 1) mit einer Zyklisierungsbindung zwischen den beiden Endcysteinen umfasst, die eine Bindungsstelle von Fertilin Beta an das Integrin der Eizelle reproduziert, für die *In-vitro-Reifung* von unreifen Eizellen.

2. Verwendung nach Anspruch 1, wobei es sich bei den Eizellen um menschliche Eizellen handelt.

3. Verwendung nach Anspruch 1 oder 2, wobei die Eizellen für einen Zeitraum zwischen 1 Minute und 4 Tagen, insbesondere bis zu 24 Stunden, in Gegenwart von 1 bis 100 µM Peptid inkubiert werden.

4. Verwendung nach einem der Ansprüche 1 bis 3, zur Verbesserung der Ploidie von Eizellen.

5. *In-vitro*-Verwendung eines zyklischen Peptids, das das zyklische Peptid der Formel C-S-F-E-E-C (SEQ ID NO: 1) mit einer Zyklisierungsbindung zwischen den beiden Endcysteinen umfasst, die eine Bindungsstelle von Fertilin Beta an das Integrin der Eizelle reproduziert, für die Kryokonservierung von Gameten und Embryonen.

## Claims

1. *In vitro* use of a cyclic peptide comprising the cyclic peptide of formula C-S-F-E-E-C (SEQ ID NO: 1) with a cyclization linkage between the two end cysteines reproducing a binding site of fertilin beta to the oocyte integrin, for the *in vitro* maturation of immature oocytes.

2. The use according to claim 1, wherein the oocytes are human oocytes.

3. The use according to claim **1** or **2,** wherein the oocytes are incubated for a period of between 1 minute and 4 days, in particular up to 24 hours, in the presence of from 1 to 100 µM of peptide.

4. The use according to one of claims **1** to **3,** for improving the ploidy of oocytes.

5. *In vitro* use of a cyclic peptide comprising the cyclic peptide of formula C-S-F-E-E-C (SEQ ID NO: 1) with a cyclization linkage between the two end cysteines reproducing a binding site of fertilin beta to the oocyte integrin, for the cryopreservation of gametes and of embryos.
